# EUROPEAN PATENT APPLICATION

(11) **EP 1 544 594 A1**
(43) Date of publication of application: **22.06.2005**
(21) Application number: 03740509.9
(22) Date of filing: 10.07.2003
(51) Int. Cl.: G01N 3/32

(54) **METHOD AND DEVICE FOR EXAMINING FATIGUE RESISTANCE OF METALLIC MATERIALS AT ULTRASONIC FREQUENCIES AND CONSTANT TEMPERATURE**

(30) Priority: 19.07.2002 ES 200201700
(71) Applicant: CONSEJO SUPERIOR DE INVESTIGACIONES CIENTIFICAS, 28006 Madrid (ES)
(72) Inventor: CAMPOS POZUELO, Cleofé Instituto de Acústica, C/ Serrano, 144 E-28006 Madrid (ES); RODR GUEZ CORRAL, Germán Instituto de Acústica, C/ Serrano, 144 E-28006 Madrid (ES); BLANCO BLANCO,Alfonso Instituto de Fisica Aplicada, C/ Serrano, 144 E-28006 Madrid (ES); ACOSTA APARICIO,Victor,M. Instituto de Acústica, C/ Serrano, 144 E-28006 Madrid (ES); MONTOYA VITINI,Fausto Instituto de Fisica Aplicada, C/ Serrano, 144 E-28006 Madrid (ES); GALLEGO JUAREZ, Juan,Antonio Instituto de Acústica, C/ Serrano, 144 E-28006 Madrid (ES)
(74) Representative: Ungria Lopez, Javier
(86) International application number: PCT/ES2003/000352
(87) International publication number: WO 2004/010112

(57) **Abstract**

The invention relates to a method and device for determining the fatigue resistance of metallic materials at ultrasonic frequencies and a constant temperature. The inventive method can be used to examine materials subjected to bending and traction/compression stresses at different temperatures ranging from atmospheric temperature to 1000 °C and at high frequency. Said method can be used to evaluate the breaking stress and the number of cycles to which the material has been subjected and to record the break profile. The device comprises an excitation system, a data acquisition system and resonant samples of the material.

## Description

A good knowledge of the fatigue resistance of metallic materials habitually used in the aeronautics and aerospace industry, in defence and in energy, is a subject of undoubted interest and topicality. In fact, there exist simple and well-established methods for determining other mechanical parameters of interest: elastic limit, breaking point, hardness, etc., but the use of a certain material in the aforementioned industries runs into the problem of fatigue breakage of its constituents at high vibration frequencies.

When the SN fatigue resistance curve is defined for carbon steels (breaking stress as a function of number of cycles) one generally refers to 10⁷ cycles and it is accepted that afterwards there exists a horizontal asymptote that allows one to talk of a fatigue limit, taking into account the value of the stress for 10⁶-10⁷ cycles. For other alloys, and in particular for those used in aeronautics (titanium alloys), the asymptote of the SN curve is not horizontal. The interest in conducting quick tests on fatigue by means of a machine for testing fatigue at ultrasonic frequencies becomes evident.

The object of this patent is the establishment of a device and a method that will permit the determination of the fatigue resistance of metallic materials at ultrasonic frequencies and at constant temperature. At ultrasonic frequencies the propagation of a crack is so fast that one will be working with the establishment of fatigue breakage thresholds for different numbers of cycles.

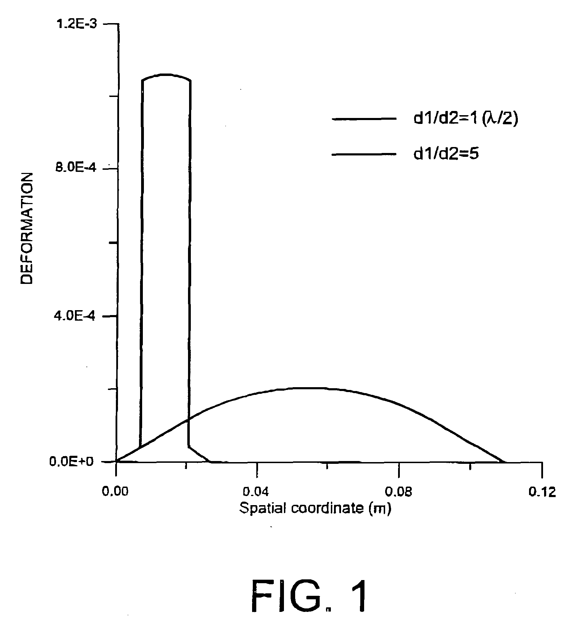

There exist other patented or published systems [1,2,3] establishing devices and/or methods for the study of ultrasonic fatigue. One of the most classical is that used by Kuzmenko [1] which works with longitudinal vibrations, it uses a magnetostrictive transducer and the test samples are resonant bars at /2 with a central groove. The vibration amplitude is controlled by means of a contact sensor. The temperature of the sample is kept constant by means of an isothermal bath. The high stresses to which the magnetostrictive transducer is subjected requires the incorporation of a cooling system for it. Document [2] patents a device which is a modification of the one just mentioned, for studying the effect of the superposition of a static force on the high frequency vibration; use is made of the same design philosophy of test samples and the same type of transducer as in [1]. The system forming the object of the present patent is based 1) on the design of resonant test samples of staggered geometry, or one which very considerably reduces the stresses of the transducer (Figure 1); 2) tests are carried out under bending and under traction/compression; 3) the breaking stress is quantified by means of direct, non-intrusive, measurement of the speed of vibration combined with a very precise calculation of stresses conducted by means of finite elements (FE): 4) a piezoelectric transducer is used for excitation of the test samples; 5) the temperature of the samples is kept constant by means of excitation by wave trains of electronically controllable length; 6) the number of cycles to which the test sample has been subjected is counted electronically; 7) when breakage occurs due to fatigue, different electronically controlled parameters undergo a sharp change (frequency and impedance), which enables the moment at which the failure occurs to be detected automatically. The device forming the object of this patent therefore consists of a simple, cheap and accurate system, with features that are clearly differentiated with regard to preceding methods and devices.

The principle of the method is based on the production, control and measurement of large amplitude (non-linear) vibrations in test samples of the material to be studied, which are excited by means of moderate or low amplitude (linear) vibrations. The applied mechanical stress is calculated using a commercial finite element code. The electronic system is controlled by computer and it counts and stores the excitation time to which the test sample has been subjected, together with the working frequency and the impedance of the transducer/sample unit. The sharp change of frequency and impedance that is produced at the moment of the break permits that moment to be detected automatically.

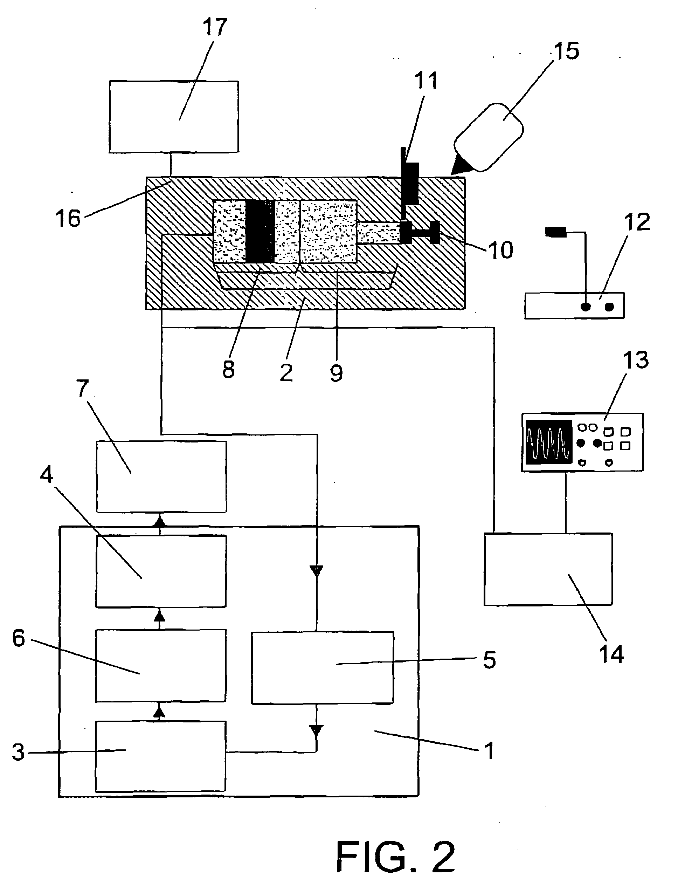

The device comprises an excitation system, a data acquisition system and the resonant samples of the material. See Figure 2.

The excitation system comprises an electronic generator (1) and a piezoelectric transducer (2).

The electronic generator used for exciting the transducer, which comprises an oscillator (3) and a power amplifier (4), is provided with a feedback system (5) for automatically adjusting the excitation frequency to the resonance frequency of the transducer. The feedback system measures the amplitude of the voltage in the transducer along with the phase difference between it and the current, making a correction in the control parameter of the oscillator frequency. The system also has a switching circuit (6) designed for producing periodical interruptions in the excitation signal; in this way, the excitation time can be controlled and so the temperature of the sample can thereby be kept constant. This switching circuit essentially consists of a programmed counter and an output stage which acts on the input signal for the power amplifier. The excitation time and the stop time can be varied between 0.1 s and 1.8 hours. With these features, it is possible to keep the temperature constant for metallic samples subjected to major strains. The device also includes a system for adaptation of impedances between the generator and the load (7) which, depending on the type of sample being considered, permits the generator to be adapted to the load and thereby obtain the maximum possible energy transfer between the two. The electronic system is endowed with a counter which informs the user of the number of wave trains applied to the sample, and the frequency and width of them, or, indeed, of the number of fatigue cycles which the test lasts for. When the electric parameters which, according to the method described above, characterise the nucleation of the fatigue crack undergo a change, the counter stops and the test is then regarded as concluded. The excitation system is digital and is connected to a personal computer which permits remote control of the different characteristic parameters of the test and the recording of the history of the break.

The excitation transducer (2), which is a resonant structure at the frequency of interest, consists of two elements of length λ/2: the piezoelectric transducer element (8) and a mechanical amplifier in staggered form (9). The transducer element is of the sandwich type consisting of four piezoelectric ceramic layers prestressed between a mass and a countermass made of steel. The mechanical amplifier is made of an alloy highly resistant to fatigue (Ti or Al 4V), it has a ratio of sections of 40/15 with a certain radius of curvature for preventing breakage due to fatigue.

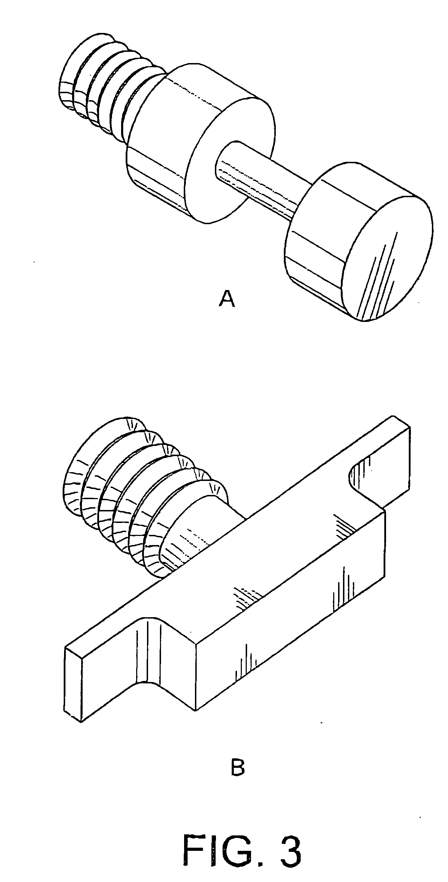

The geometry of the samples (10) used is fundamental for the success of the fatigue system. The test samples are designed so that, first of all, they resonate at the frequency of the transducer and, secondly, so that there is a large concentration of energy in them. In fact, the system has been designed for studying materials that are especially resistant to fatigue, which means that there is a risk of the transducer breaking before the sample does if the latter is not correctly designed. So, samples with a staggered geometry have been designed and constructed. For the traction/compression tests cylindrical samples with a staggered profile as shown in Figure 3 (A) are used. Figure 1 shows the amplitude of strain in a staggered test sample (A) with ratio of diameters (*d*_{*1*}/*d*_{*2*} = 5) compared to a cylinder, and it can be seen that with this type of geometry very high strains in the material can be achieved without large excitation amplitudes, in other words, in the linear range of the excitation transducer (the maximum strain in the transducer would coincide with the strain in a test sample of length λ/2). The samples are constructed in such a way that they are resonant at the frequency of the transducer. It can be confirmed that the resonance condition for this type of geometry is *tan(ωl*/*4c)* = *d*_{*2*}/*d*_{*1*}, where ω is the resonance frequency, *l* the length of the sample, c the speed of sound in the material being studied, and *d*_{*2*} and *d*_{*1*} are the diameters of the sections of the bar [4]. It can be seen that, owing to the special geometry of the samples, the resonance lengths are less than λ/2, which results in a greater concentration of energy and, therefore, as has been stated, in high strain amplitudes (Figure 1). This permits the test samples to break under fatigue while keeping the transducer working in its linear range, even when dealing with materials that are highly resistant to fatigue as in the case of the same material as that used for the mechanical amplifier of the transducer.

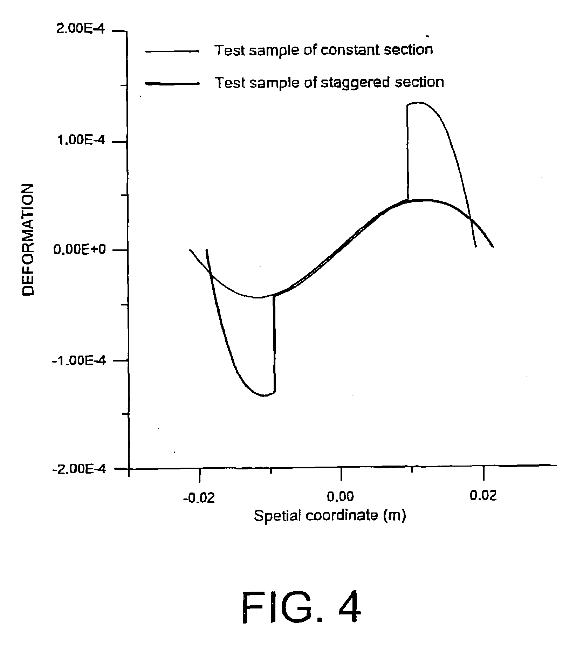

For the bending vibration tests, the same principle is followed, and prismatic test samples of staggered profile (11) are designed and constructed, joined via their central part (in this case thicker) to the excitation transducer (Figure 3 B). Figure 4 shows the comparison of the distribution of strains for a sample of constant section and another of staggered section [5]. Indeed, that stated for samples vibrated extensionally is also evident for this type of vibration.

The samples are screwed to the transducer at a point of minimum stress and they vibrate freely, without any point of contact.

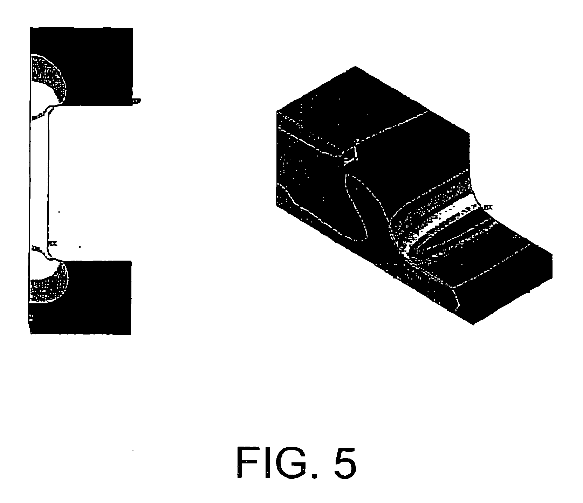

The models used in figures 1 and 4 are analytical models which do not take into account effects such as the concentrations of stresses in the changes of section. In order to correctly evaluate the stresses to which a test sample is subjected starting from the measurement of the amplitude of the speed of vibration at a point, a numerical calculation is performed (using a commercial finite element code) of the distribution of stresses in the samples. Figure 5 shows the results corresponding to two types of sample used in the fatigue tests.

The data acquisition system has the fundamental characteristic of using non-intrusive methods. This feature is essential when working at high vibration amplitudes, where any contact would cause major local heating and the consequent temperature gradients which have a large effect on the characteristics of the material being studied and which accelerate the fatigue processes. So, the amplitude of displacements is measured by means of a vibratometer based on a He-Ne laser interferometer (12). The vibratometer measures frequencies up to 1.5 MHz and vibration speeds of between 10 µm/s and 10 m/s. The vibratometer is connected to a digital oscilloscope (13) and this to a PC (14) where the signals are stored and analysed by standard FFT methods.

The system permits tests to be conducted with temperature controlled by means of a ceramic oven thermally insulated and heated, provided with a control system (17) which keeps the temperature inside the oven constant. This temperature is measured by means of a thermocouple and is regulated by a multifunction controller connected to the heating resistances for tests up to 1000 °C. The multifunction controller (17) is connected to the PC where the temperatures are recorded and the temperature for carrying out the test is defined. The surface temperature of the test sample is measured by means of an infrared point temperature meter (15) without having any contact in the vibration node of the sample, which is the point of maximum heating, and where heating of equal to or greater than 1 °C at that point is not permitted.

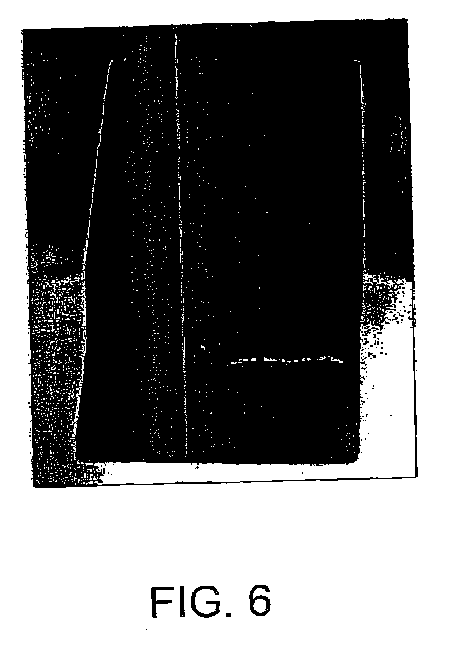

By way of example, in figure 6 we show a test sample for bending vibration broken by ultrasonic fatigue by means of the method and device described herein.

### REFERENCES

1. V. A. Kuzmenko, "Fatigue strength of structural materials at sonic and ultrasonic loading frequencies" Ultrasonics 13 (1) 21-30 (1975)
2. C. Bathias, A. Billmann, Tieying W., "Système d'assai pour fatigue vibratoire à charge moyenne non nulle" publication No. 2 680 003 (1993)
3. Fukihara Mikio, "Ultrasonic vibration type fatigue tester" publication No. JP7035668 (1995)
4. C. Campos-Pozuelo and J.A. Gallego-Juárez, "Limiting strain subjected to high-intensity ultrasound" 82 823-828 (1996)
5. C. Campos-Pozuelo and J.A. Gallego-Juárez, "Finite amplitude: flexural vibrations at ultrasonic frequencies in metallic bars", J. Acoust. Soc. Am. 98 1742-1750 (1995)

## Claims

1. Method and device for determining the ultrasonic fatigue resistance of materials **characterised by** the production, control and measurement of large amplitude (non-linear) vibrations in test samples of the material to be studied which are excited by means of moderate or low amplitude (linear) vibrations.

2. Method and device for determining the ultrasonic fatigue resistance of materials **characterised by** the use of a piezoelectric transducer with a mechanical amplifier of staggered section and of test samples vibrating under bending or extensionally, of staggered section, resonant at the frequency of interest.

3. Method and device for determining the ultrasonic fatigue resistance of materials according to the above claims **characterised by** the use of an electronic excitation system permitting work to be carried out at all times at the resonance frequency of the transducer/sample system and at constant temperature, by means of excitation of wave trains of controlled length.

4. Method and device for determining the ultrasonic fatigue resistance of materials according to the above claims **characterised by** the use of a data acquisition system which has no point of contact, without affecting the resonance of the test sample, nor the distribution of stresses or temperature.

5. Method and device for determining the ultrasonic fatigue resistance of materials according to the above claims **characterised by** being equipped with a ceramic oven permitting the conducting of tests at different temperatures from atmospheric temperature up to 1000 °C.

6. Method and device for determining the ultrasonic fatigue resistance of materials according to the above claims **characterised by** being controlled by computer which permits the recording of the history of the break, the automatic control of the system parameters and, as a consequence, the exact knowledge of the moment of the break without the operator being present.
